# EUROPEAN PATENT APPLICATION

(11) **EP 3 982 323 A1**
(43) Date of publication of application: **13.04.2022**
(21) Application number: 20200564.1
(22) Date of filing: 07.10.2020
(51) Int. Cl.: G06T 7/00, G06T 7/136, G06T 7/62, G01N 15/05, G01N 33/49

(54) **SYSTEM AND METHOD FOR QUANTIFYING A PHASE IN A DISPERSION**

(71) Applicant: Medela Holding AG, 6340 Baar (CH)
(72) Inventor: Jäger, Linda, 8049 Zürich (CH)
(74) Representative: Grünecker Patent- und Rechtsanwälte PartG mbB

(57) **Abstract**

The invention provides a system for quantifying the volume fraction of a first phase in a dispersion, comprising a vessel for containing a sample of the dispersion, a centrifuge for centrifuging the vessel containing the dispersion, a computer program comprising instructions which, when the program is executed by a computer, cause the computer to carry out an image analysis algorithm on a digital photo obtained from the vessel after centrifugation to determine the size of the first phase and the size of the entire sample in the vessel. This information allows to calculate the volume fraction of the first phase from the ratio of the size of the first phase to the size of the entire sample in the vessel. The invention further provides a method for quantifying the volume fraction of a first phase in a dispersion, preferably for measuring the hematocrit and/or the creamatocrit, comprising providing a sample of the dispersion in a vessel and centrifuging the vessel to separate the different phases of the dispersion. The method further involves obtaining a digital photo of the sample after centrifugation and performing a computer-implemented image analysis algorithm on the digital photo to determine the size of the first phase and the size of the entire sample in the vessel and to calculate the volume fraction of the first phase from the ratio of the size of the first phase to the size of the entire sample in the vessel.

## Description

The present invention relates to a system and a method for quantifying the volume fraction of a phase in a dispersion following phase partitioning. The invention particularly relates to a system and a method for measuring the hematocrit of human blood and/or the creamatocrit of human milk.

### Technical background

The quantification of different phases of a dispersion in terms of their volume fraction is an important tool in the analysis of biological fluids. It is usually performed by quantitative centrifugation to separate the different phases of the dispersion and determining their relative volume ratios.

This method is applied, for example, for determining the volume fraction of red blood cells in blood (the hematocrit value). Blood is a suspension of blood cells in a fluid medium. The hematocrit value measures the packed cell volume (PCV) or erythrocyte volume relative to whole blood. The hematocrit value can also be determined experimentally by spectrophotometry or measured by blood-gas analyzers or inferred from hemoglobin values. A major method for measuring the hematocrit value is by centrifuging EDTA-treated or heparinized venous or finger/heel prick blood in a capillary tube to separate the red blood cells from the liquid blood plasma and measuring the volume of packed red blood cells relative to the volume of the blood sample. Since a capillary tube with defined dimensions is used, the volumetric ratios of the separated layers in the tube can be determined.

The applications of hematocrit analysis are many. A lower than normal hematocrit can indicate a low red blood cell count (anemia/blood loss), a large number of white blood cells (long-term illness/leukemia/lymphoma), vitamin or mineral deficiencies, whereas a higher than normal hematocrit can indicate dehydration, polycythemia vera or lung-and heart diseases. However, normal values vary with gender, race and age. In addition, hematocrit measurements are important for diabetes patients since hematocrit values can interfere with correct measurements of blood glucose using POC (point of care) self-mentoring blood glucose meters/devices. Hematocrit measurements are therefore included or corrected for in blood glucose meters. Moreover, the capillary method is also used, for example, to diagnose tropical diseases such as for example dengue fever and malaria, following pre-treatment of serological/blood samples. Moreover, anemia is prevalent in pregnancy with up to 60% of pregnant women in resource- poor settings having iron deficiency anemia, a condition known to impact outcomes of both mother and infant, including increased risk for sepsis, maternal mortality, prenatal mortality and low infant birth weight.

Another application of quantitative centrifugation is the measurement of the creamatocrit value of human milk. Milk is an emulsion of an aqueous phase and a hydrophobic cream phase. The creamatocrit is the volume percentage of the cream phase in human milk. The creamatocrit method i.e. the quantification of cream content in raw breast milk was invented in the mid 1970-ies. It serves to determine the lipid and caloric content in mothers' milk. Creamatocrit readings can help with lactation management, individualized nutrition in neonatal intensive care units, human milk dose estimations, provide milk sample processing information, estimate breast pump efficiency etc.

Similarly to the hematocrit, the creamatocrit can be determined by capillary centrifugation of milk to separate the cream phase from the hydrophilic phase and measuring the height of the different layers in the capillary. An example of such a method is disclosed in Lucas et al (A Lucas et al., "Creamatocrit: simple clinical technique for estimating fat concentration and energy value of human milk", British Medical Journal, 1978, 1, 1018-1020). Lucas et al disclose the separation of the cream phase from milk by capillary centrifugation and the subsequent measurement of the height of the cream layer using Vernier calipers or a microscope incorporating a moving stage with a Vernier scale.

A centrifuge and a method for measuring the creamatocrit is also disclosed by EP 1 617 213 A1. The centrifuge comprises a fixed angle rotor for holding a capillary and a reader for measuring the height of the layers in the capillary after centrifugation. The reader comprises a movable marker that can be moved manually to the positions of the interfaces between different phase in the capillary, such as the interface between an aqueous milk layer phase and a cream phase. By electronically determining the position of the marker at each interface, the relative volume of the cream phase can be calculated.

The centrifuge according to EP 1 617 213 A1, however, is expensive, laborious and suffers from high errors due to the method which results in high inter- and intra-user variability. The fixed angle rotor gives a slanting of the capillary meniscus resulting in additional reading inaccuracies. Moreover, upon repeated operation, the centrifuge heats up and partially "melts" the cream, causing it to separate into a yellow low molecular weight lipid phase and a "dry" cream/solids phase introducing an additional reading bias.

A general challenge when measuring the creamatocrit is that the cream content in milk can be as low as 0.8%, potentially corresponding to a sub millimeter thin meniscus, depending on the capillary diameter and the sample volume. Such a thin meniscus cannot be reliably determined by conventional methods. Conventional methods, such as the above-described device of EP 1 617 213 A1, cannot measure below 1% cream and suffer from standard deviations of 25% in the range of 1-1.5% cream, making the reader erroneous and suboptimal for human milk samples of low cream content. The cream content in human milk is variable and since the low cream content is also used indirectly to determine, for example, pump efficiency, large measurement errors in this range can bias the end results.

Similar difficulties apply to the measurement of the hematocrit value, which is typically done by a hematocrit slider/card/reader. Due to these difficulties, the measurements usually have to be carried out by skilled workers who are trained in operating the required measuring devices to reduce the measurement error.

The manual operation and inspection required for reading single capillaries further reduces the throughput and thus increases the cost of current methodologies.

### Technical problem

The present invention aims at providing an improved system and a method for quantifying the volume fraction of different phases of a dispersion. The system and method should reduce the measurement error, particularly in cases where the volume fraction is relatively small. Preferably, the system and method should be suitable for determining the hematocrit of human or animal blood and/or the creamatocrit of human or animal milk. Moreover, using the system or carrying out the method should not require special training so that the system and method can easily be used by the average user instead of specially trained workers.

This problem is solved by the system and the method disclosed herein.

### Summary of the invention

In a first aspect, the invention relates to a system for quantifying the volume fraction of a first phase in a dispersion, comprising
a vessel for containing a sample of the dispersion,
a centrifuge for centrifuging the vessel containing the dispersion,
a computer program comprising instructions which, when the program is executed by a computer, cause the computer to carry out an image analysis algorithm on a digital photo obtained from the vessel after centrifugation.

According to a preferred embodiment, the image analysis algorithm determines the size of the first phase and the size of the entire sample in the vessel and calculates the volume fraction of the first phase from the ratio of the size of the first phase to the size of the entire sample in the vessel.

According to a preferred embodiment, the system further comprises a digital camera for obtaining a digital photo of the sample after centrifugation, the digital camera having an electronic interface for transferring the digital image to a computer that executes the computer program.

According to a preferred embodiment, the system further comprises a sample holder for holding the vessel during centrifugation or for obtaining a digital photo of the sample after centrifugation According to a preferred embodiment, the sample holder has at least one surface that is of a contrasting color to the color of the first phase.

According to a preferred embodiment, the vessel is formed by a microfluidic channel on the sample holder.

According to a preferred embodiment, the sample holder is configured to hold the vessel horizontally relative to the centrifugation axis during centrifugation.

According to a preferred embodiment, the centrifuge is a hand-powered centrifuge.

According to a preferred embodiment, the centrifuge is a fidget-spinner comprising two or more lobes attached to a ball bearing, the lobes being configured to rotate around the rotational axis of the ball bearing and at least one of the lobes being configured to hold the vessel during centrifugation.

According to a preferred embodiment, the centrifuge comprises a disk-shaped rotor for holding the vessel and one or more strings, the strings passing through two or more through holes disposed on the disk-shaped rotor.

In another aspect, the invention relates to a method for quantifying the volume fraction of a first phase in a dispersion, comprising
providing a sample of the dispersion in a vessel;
centrifuging the vessel to separate the different phases of the dispersion;
obtaining a digital photo of the sample after centrifugation;
performing a computer-implemented image analysis algorithm on the digital photo to determine the size of the first phase and the size of the entire sample in the vessel;
and calculating the volume fraction of the first phase from the ratio of the size of the first phase to the size of the entire sample in the vessel.

According to a preferred embodiment, the dispersion is an animal or human body fluid.

According to a preferred embodiment, the dispersion is animal or human blood and the first phase comprises red blood cells or the dispersion is animal or human milk and the first phase comprises cream.

According to a preferred embodiment, the vessel is centrifuged using a hand-powered centrifuge.

According to a preferred embodiment, the vessel is placed horizontally relative to the centrifugation axis during centrifugation.

According to a preferred embodiment, the vessel is placed on a surface that is of a contrasting color to the color of the first phase for obtaining the digital photo of the vessel.

### Technical effects of the invention

The method and system according to the invention allow for the measurement of volume fractions of individual phases in dispersions with high accuracy. By taking a digital photo of the sample after centrifugation and analyzing the photo by an image analysis algorithm, the position of the interfaces between different layers in the vessel and thus the respective size of the layers can be more accurately determined than by conventional methods such as calipers, rulers, microscopes or creamatocrit readers. In a preferred embodiment, the volume fraction can be determined with an accuracy of ± 0.3 %. A particular advantage of the present invention is that the digital photo can be acquired using conventional digital cameras, such as the cameras included in modern smartphones. This further enhances the application of the invention since worldwide smartphone penetration rates are at 45% (2020) and rising. Moreover, no special training is required to carry out the measurement, as the analysis of the digital photo is performed by an algorithm.

### Description of the drawings

Fig. 1 Front view of a disk-shaped rotor for a hand-powered whirligig centrifuge.
Fig. 2 Front view of an alternative embodiment of the disk-shaped rotor.
Fig. 3 Front view of an alternative embodiment of the disk-shaped rotor.
Fig. 4 Drawing of a disk-shaped rotor according to Fig. 2 attached to a pair of strings.
Fig. 5 Drawing of a disk-shaped rotor according to Fig. 1 attached to a pair of strings.
Fig. 6 Schematic drawing of the rotation mechanism of the rotor according to Figs. 1 to 5.

### Detailed description of the invention

The method disclosed herein relates to the measurement of the volume fraction of a first phase of a dispersion. Within the context of the present disclosure, the term "dispersion" refers to a composition in which particles of one material (the dispersed phase) are dispersed in a continuous phase of another material (the dispersion medium). The particle size and size distribution of the dispersed phase are not particularly limited. The dispersion may contain more than one dispersed material, i.e. it may contain different dispersed phases. The kind of dispersion that can be analyzed by the method is not particularly limited, as long as at least a first phase can be separated by centrifugation from the remaining components.

The method allows to determine the volume fraction of at least one phase of the dispersion. This phase is referred to as "the first phase" in the context of this invention. The first phase is component of the dispersion that can be separated from the rest of the dispersion by centrifugation. The first phase may be the dispersion medium or any phase dispersed therein. The first phase may also be a component of the dispersion medium, in case the dispersion medium can be separated by centrifugation into individual components.

Further, the method may also allow determining the volume fraction of additional phases of the dispersion, as long as these additional phases can be separated by centrifugation.

Preferably, the dispersion is an emulsion (a dispersion of a liquid material in a liquid medium) or a suspension (a dispersion of a solid material in a liquid medium).

In a preferred embodiment, the dispersion contains biological components, for example a cell suspension or an emulsion of biological compounds. More preferably, the dispersion is an animal or human body fluid, such as milk or blood. Even more preferably, the dispersion is milk or blood, most preferably human milk or human blood. The body fluid may be treated by the addition of further components before being analyzed by the method. For example, the dispersion may be diluted or further compounds may be added that assist in phase separation. In the case of blood, for example, the blood may preferably be treated by addition of anticoagulants, such as EDTA or heparin. The addition of anticoagulants to blood is preferred, as it prevents coagulation of blood components during the centrifugation step, which might otherwise negatively affect the measurement result.

In a preferred embodiment, the dispersion is blood and the first phase that is separated by centrifugation contains the red blood cells (erythrocytes). The volume fraction of the first phase that is measured by the method thus corresponds to the hematocrit. In a likewise preferred embodiment, the dispersion is milk and the first phase contains the cream fraction. In this case, the method measures the creamatocrit.

During centrifugation and the subsequent image acquisition, the dispersion is preferably kept at a temperature, where no precipitation of components occurs. This means keeping the dispersion at a temperature not lower than a given precipitation temperature. Likewise, the temperature should not be so high as to induce denaturation or phase separation of certain components, which might otherwise also lead to an unwanted input for image analysis. Improper handling could otherwise distort the measurement results and lead to an over- or underestimation of the volume fraction of the first phase, depending on which components precipitate. This is particularly important for dispersions containing biological components. The exact temperature limits depend on the nature of the dispersion. Preferably, the dispersion is analyzed at a temperature from 5°C to 45°C, preferably 10°C to 40°C, more preferably from 20°C to 30°C. If the dispersion is blood, the temperature is preferably kept at 5°C to 15°C.

The dispersion is preferably provided in a vessel that allows for a clearly visible separation of the first phase from the rest of the dispersion during centrifugation. The vessel preferably is made of a transparent material, such as glass or plastic, or it may contain a transparent window to allow imaging of the separated dispersion after centrifugation. The vessel preferably has a cylindrical shape with a circular cross-section. The vessel may have a constant diameter along its longitudinal axis so that the height of each layer after separation of the dispersion is proportional to its volume. The diameter may, however, also vary, as long as the variation of the diameter is known. For example, the vessel may be tapered and have a decreasing inner diameter. In case the diameter varies along the longitudinal axis of the vessel, that variation may be accounted for by the image analysis algorithm used to measure the size of the separated layers. In a preferred embodiment, the vessel is a capillary.

The inner cavity of the vessel preferably has a length of 20 mm to 100 mm and a diameter of 0.2 mm to 5 mm. The inner cavity refers to the opening of the vessel, which holds the dispersion. In a preferred embodiment, the inner cavity has a length of 30 mm to 50 mm and a diameter of 0.5 mm to 2 mm.

In a preferred embodiment, the vessel is formed by a microfluidic channel on a sample holder as described below.

The dispersion is centrifuged to separate at least the first phase from the rest of the dispersion. Thereby, different layers of the separated dispersion form in the vessel that can be distinguished in the digital photo and the size of each layer can be measured to determine the volume fraction of the first phase. In addition, the method may also be used to separate additional phases from the dispersion and determine their volume ratio.

To prevent leakage of the dispersion, the vessel is preferably sealed at one end, for example, by a piece of clay.

Centrifugation is performed using a suitable centrifuge, for example a bench-top or hand-held centrifuge. Preferably, the centrifuge is a hand-held device, such as one of the hand-held centrifuges described below.

Centrifugation is preferably performed with a relative centrifugal force of at least 5,000 g, preferably at least 10,000 g, more preferably at least 12,000 g, most preferably at least 20,000 g. In a preferred embodiment, the relative centrifugal force is 10,000 g to 30,000g. The centrifugation preferably is 1 minute to 20 minutes, more preferably 3 minutes to 10 minutes.

During centrifugation, the vessel can be placed at a fixed angle relative to the centrifugation axis. For example, the vessel can be placed at an angle between 20° and 90° relative to the centrifugation axis, more preferably 40° to 90°, even more preferably 75° to 90° . Most preferably, the vessel is placed horizontally, i.e. at a substantially right angle to the centrifugation axis. Placing the vessel horizontally has the advantage of producing a horizontal meniscus at the interface between different phases of the dispersion. A horizontal meniscus is horizontal relative to the long axis of the vessel. This facilitates image analysis of the separated layers. Placing the vessel at an angle of less than 90° during centrifugation results in a slanted meniscus. This would make it more difficult to analyze the meniscus manually, however, it can be accounted for by adjusting the image analysis algorithm accordingly.

After centrifugation, a digital photo is acquired of the vessel containing the separated dispersion. The digital photo may be acquired using any type of digital camera. Preferably, the vessel is placed orthogonally and centrally relative to the optical axis of the digital camera to avoid any measurement artifacts due to optical distortions.

Preferably, the vessel is placed on a surface that acts as a background when acquiring the digital photo. The surface may be the surface of a dedicated sample holder, such as a tray on which the vessel is placed. The surface may also be marked with fiducial markers, which are also recorded on the digital photo. These markers can be used by the image analysis algorithm to correct for optical distortions, for example, distortions produced by placing the vessel not perfectly orthogonal to the optical axis, i.e. at an angle of less than 90° relative to the optical axis of the digital camera. In a further embodiment, the surface may be marked with an identification tag, preferably a machine-readable tag such as a QR code, to allow identification of the sample by electronic data processing means.

In a preferred embodiment, the vessel is placed on a surface that is of contrasting color to the color of the dispersion. For example, if the dispersion is milk, which has a relatively light color, the surface preferably has a dark color, such as dark gray, dark blue, or black. If the dispersion is blood, which has a red color, the surface preferably has a light color, such as light blue, light grey, or white. Using a surface having a contrasting color facilitates the image segmentation used by the image analysis algorithm to identify the location of the vessel in the digital photo.

To facilitate image analysis, the vessel is preferably placed on a surface having a single, homogeneous color, apart from the optional fiducial markers and identification tags mentioned above.

The digital photo is transferred to a computer that is configured to carry out the image analysis algorithm.

The image analysis algorithm preferably comprises a step of correcting for optical distortions. The image analysis algorithm preferably further comprises a step of image segmentation to separate the image pixels belonging to the background from the image pixels belonging to the vessel. This step may also be used to categorize the sample chamber for it to be recognized. The image analysis algorithm preferably comprises an additional step of image segmentation to identify the pixels belonging to the interfaces between different layers of the separated dispersion and/or to separate the pixels belonging to the different layers. The image segmentation steps preferably use a threshold algorithm to distinguish background pixels and/or an edge detection algorithm to identify the potentially numerous vessels and/or the different layers in the vessels

The image analysis algorithm preferably comprises a step of determining the size of the different layers in the vessel. The image analysis algorithm at least determined the size of the first phase and the size of the entire sample in the digital photo. To determine the size of the respective layers, the algorithm may determine, for example, their area or their height. The height is measured along the longitudinal axis of the vessel. In a preferred embodiment, the vessel has a uniform width along its longitudinal axis so that the height of each layer is proportional to each layer's volume. Therefore, the image analysis algorithm preferably determines the height of each layer. The image analysis algorithm may also determine the area of each layer based on the number of pixels that are associated with each layer.

The image analysis algorithm may further comprise a step of calculating the ratio of the size of the first phase to the size of the entire dispersion in the vessel to obtain the volume fraction of the first phase. If the vessel does not have a uniform diameter, the shape of the vessel and the variation in the diameter are taken into account by the image analysis algorithm to calculate the volume fraction. The image analysis algorithm may also comprise additional steps to calculate the volume fraction of further phases of the dispersion relative to the entire dispersion. The image analysis algorithm does not necessarily have to calculate the ratio of the sizes of the different phases of the dispersion. Instead, the image analysis algorithm may also only provide the size of each phase identified in the image. This information can then be processed either by an additional algorithm or by the user.

The image analysis algorithm is preferably an automatic image analysis algorithm, which performs the abovementioned steps without requiring additional user input.

While the above description refers to the analysis of a single vessel, it is also possible and preferable to image and analyze more than one vessel in parallel. For example, it is possible to centrifuge two or more vessels, place all vessels next to each other on the same surface and image all vessels simultaneously. This produces a single digital photo showing two or more vessels, which can be analyzed by the image analysis algorithm, yielding the volume fraction of each first phase in each vessel. Each vessel in this case may contain a sample from the same dispersion so that several parallel measurements of the volume fraction of the first phase can be made simultaneously. This improves the measurement accuracy. Alternatively, each vessel contains a sample of a different vessel, allowing to measure the volume fraction of the first phase in different test subjects. Thereby, parallel imaging increases throughput of the measurement method. Parallel imaging may also be used to train the image algorithm with a large number of images of dispersions each having a known volume fraction of the first phase.

The system disclosed herein comprises a vessel, a centrifuge to separate the dispersion, and a computer program to analyze the volume fraction of a first phase in the separated dispersion. This system allows the user to carry out the method of the present disclosure.

The system does not necessarily have to contain a digital camera. This camera can instead be provided by the user. For example, the user can provide a digital camera that is part of modern smartphones to acquire a digital photo of the sample after centrifugation. Alternatively, the system may also contain a digital camera for obtaining a digital photo of the sample after centrifugation. The digital camera must have an electronic interface for transferring the digital image to the computer that executes the computer program or alternatively have an application for data/image analysis.

The computer program is meant to be executed on a computer. When the computer program is executed on a computer, the computer carries out the image analysis algorithm described above.

The computer program is preferably stored on a computer, such as a mobile electronic device, a desktop computer or a server. The computer preferably comprises at least one electronic processor and at least one storage unit to execute the computer program. The computer may be part of the same device as the digital camera, such as a smartphone. Alternatively the computer may be a separate device to which the digital photo is transferred. For example, the computer can be a server, to which the digital image can be transferred over a network connection, or a desktop computer to which the digital photo is transferred. The computer may also be distributed system comprising, for example, a client device, such as a smartphone, having an interface for receiving the digital photo from a digital camera and an interface for transferring the digital photo to a server over a network connection. The server may be configured to execute the computer program, so that the image analysis algorithm is substantially carried out on the server. The results of the calculation may be transferred back to the client device, which preferably has a display device for displaying the results of the calculation to the user.

The system preferably further comprises a sample holder for holding the vessel. The sample holder may preferably be configured to hold the sample during centrifugation and/or the subsequent image acquisition. Thereby, the sample holder may stabilize the position of the vessel during centrifugation and/or image acquisition. Preferably, the sample holder has a dual function by holding the vessel during centrifugation and acting as a surface to hold the vessel for taking the digital photo of the sample after centrifugation.

The sample holder is preferably made from plastic, particularly from ABS, MABS, PMMA, paper, cardboard (carton), other sustainable materials like bamboo or any combination thereof. If the sample holder is used to hold the vessel during centrifugation, PMMA is a preferred material, due to its high resistance to frictional heat produced during centrifugation.

In one embodiment, the system comprises a sample holder for holding the vessel during centrifugation. Preferably, the sample holder is configured to hold the vessel at a fixed angle relative to the centrifugation axis during centrifugation, preferably an angle of 90°. The sample holder may be disk-shaped, for example.

Preferably, the sample holder comprises a coupling configured to attach the sample holder to the rotor or the drive shaft of the centrifuge. The sample holder may be attached removably or fixedly to the rotor or the drive shaft. Preferably, the sample holder is removably attached to the rotor or the drive shaft and can be attached and removed manually, without the need for additional tools.

The sample holder may further comprise one or more fixing means configured to attach one or more vessels. Preferably, the vessels are clipped into the fixing means. Thereby the vessels may be attached to the sample holder without the need for additional tools. The vessels are preferably attached removable to the fixing means.

In a preferred embodiment, the vessels may be formed by channels integrally formed in the sample holder. Preferably, the sample holder comprises microfluidic channels. The channels formed in the sample holder comprise at least one transparent window through which the dispersion inside the channels can be observed. The channels and/or the sample holder may also be fully formed from a transparent material.

Alternatively, the vessel may also be attached directly to the centrifuge rotor.

In one embodiment, the sample holder may provide a surface that acts as a background when acquiring the digital photo. This may be the same sample holder as the sample holder for centrifugation described above. It may also be a separate sample holder, such as a tray, to which the vessel is transferred after centrifugation. If the sample holder also serves to hold the vessel during centrifugation, this allows to take the digital photo without having to remove the vessel from the sample holder and preferably also without having to remove the vessel from the centrifuge. The sample holder preferably provides a surface having a contrasting color to the color of the dispersion, as explained above. In a preferred embodiment, the sample holder provides at least two differently colored surfaces to provide contrasting backgrounds for different kind of dispersions. For example, the sample holder may have a first surface with a light color, such as light blue, light grey or white, and a second surface with a dark color, such as dark grey, dark blue or black.

The centrifuge may be, for example, a bench-top centrifuge or a hand-held centrifuge. The centrifuge may be powered by an electrical motor or may be hand-powered. The centrifuge preferably achieves a relative centrifugal force of at least 5,000 g, preferably at least 10,000 g, more preferably at least 12,000 g, most preferably at least 20,000 g. In a preferred embodiment the relative centrifugal force is 10,000 g to 30,000 g.

Preferably, the centrifuge is a hand-powered centrifuge. Such a centrifuge can be produced at low cost and thus offers a cheap solution for decentralized point-of-care diagnostic applications in resource-poor settings. Further, a hand-powered centrifuge does not require connection to a power supply and does not require a trained operator. It can, therefore, be easily operated by the end user. A hand-powered centrifuge is particularly preferable for the analysis of blood or milk, which can be separated at a relatively low relative centrifugal force. An example for a hand-powered centrifuge for isolating blood plasma is disclosed by A. P. Wong et al. (A. P. Wong et al., "Egg beater as centrifuge: isolating human blood plasma from whole blood in resource-poor settings", Lab Chip, 2008, 8, 2032-2037).

In a preferred embodiment, the centrifuge is a fidget spinner. A fidget spinner is characterized in that it comprises two or more lobes attached to a ball bearing. The lobes act as a centrifuge rotor that spins around the rotational axis of the ball bearing. The fidget spinner can be held in one hand and can be set into motion by manually flipping the lobes. Examples for such a centrifuge are disclosed by I. Michael et al. (I. Michael et al., "A fidget spinner for the point-of-care diagnosis of urinary tract infection", Nature Biomedical Engineering, 2020, 4, 591-600) and by C.-H. Liu et al. (C.-H. Liu et al., "Blood plasma separation using a fidget-spinner", Anal. Chem., 2019, 91, 1247-1253). At least one of the lobes is configured to attach to one or more vessels holding the dispersion.

In another embodiment, the centrifuge is based on a whirligig, a circular disc spun by pulling on strings passing through its center. Examples for such a centrifuge are disclosed in WO 2017/127248 A1 and by M. S. Bhamla et al. (M. S. Bhamla et al., "Hand-powered ultralow-cost paper centrifuge", Nature Biomedical Engineering, 2017, 1, Article number 0009). Such a centrifuge is characterized in that it comprises a disk-shaped rotor configured for holding the vessel and two or strings passing through two or more through holes disposed on the disk-shaped rotor. The strings extends substantially orthogonal to the plane of the rotor. The rotor is actuated by successive and repeated winding and unwinding of the strings. In the unwinding phase, a force applied by pulling opposite ends of the strings outwards accelerates the disk-shaped rotor to its maximum rotational speed. In the winding phase, the inertia of the disk-shaped rotor rewinds the strings, drawing the opposite ends of the strings back inwards. Preferably, the disk-shaped rotor in this embodiment is formed by the sample described above. Preferably, the disk-shaped rotor forms one or more channels, such as microfluidic channels, for holding the dispersion as described above for the sample holder. More preferably, the disk-shaped rotor also acts as a sample holder during the subsequent image acquisition.

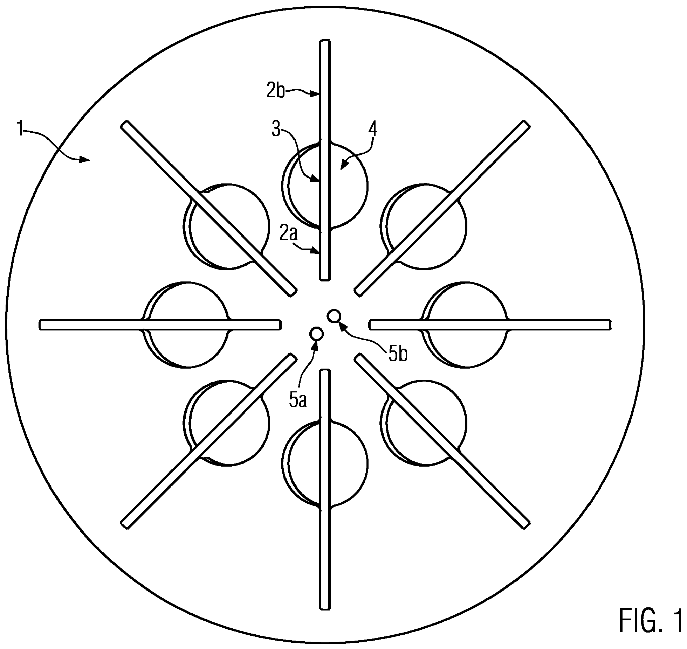

Exemplary embodiments of a hand-powered whirligig centrifuge are illustrated in Figures 1 to 6.

Figure 1 shows an exemplary embodiment of a disk-shaped rotor 1 for a hand-powered whirligig centrifuge. The body of the disk-shaped rotor 1 is preferably made from a single piece of material, such as injection-molded plastic. The material may, for example, be opaque or transparent. The rotor 1 contains several pairs of slots 2a, 2b for holding sample vessels 3. In the exemplary figures, the vessels are shown to be capillaries, however, other shapes of vessels are also possible. Each vessel is clipped into a pair of slots 2a, 2b and is thus removably attached to the disk-shaped rotor. The vessels are thereby held horizontally relative to the rotational axis, which passes through the center of the disk-shaped rotor 1. The exemplary rotor has eight pairs of slots and can take up to eight vessels. It is also possible to vary the number of pairs of slots to provide a rotor for more or less vessels. Each pair of slots 2a, 2b comprises a proximal slot 2a, which is located close to the center of the disk-shaped rotor 1, and a distal slot 2b, which is located closer to the outer circumference of the disk-shaped rotor 1. The proximal and distal slots 2a, 2b are separated by a circular recess 4. The recess 4 allows the user to grasp the vessel 3 in order to easily remove the vessel 3 from the rotor 1. The recess 4 may have the form of a well with a closed bottom part, as shown in Figure 1. Alternatively, the recess may be replaced with a circular cutout in the body of the rotor. The rotor 1 further comprises two through holes 5a, 5b, which are arranged on opposite sides of the central rotational axis.

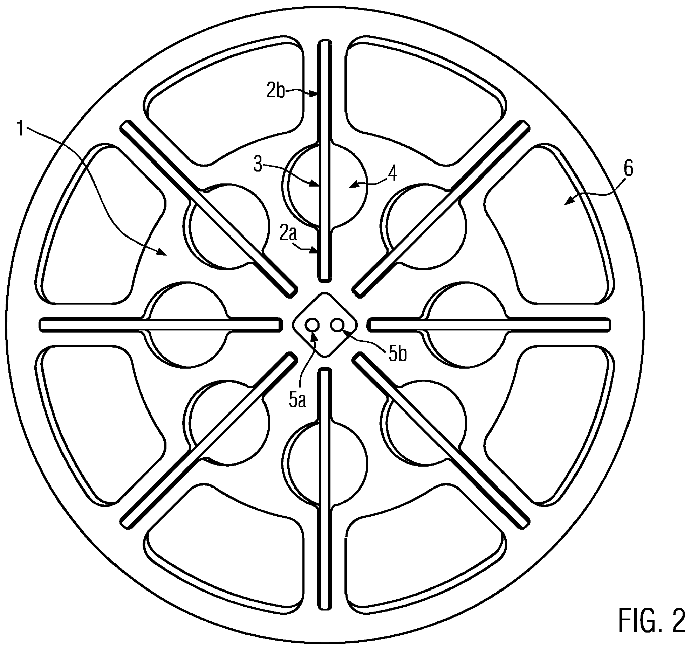

Figure 2 shows an alternative embodiment of the disk-shaped rotor 1. The rotor of Figure 2 has the same features as that of Figure 1 and has additional cutouts 6 in the disc. The cutouts 6 are arranged distally from the central rotational axis. These cutouts reduce the weight of the rotor so that less force is required to accelerate the rotor during centrifugation.

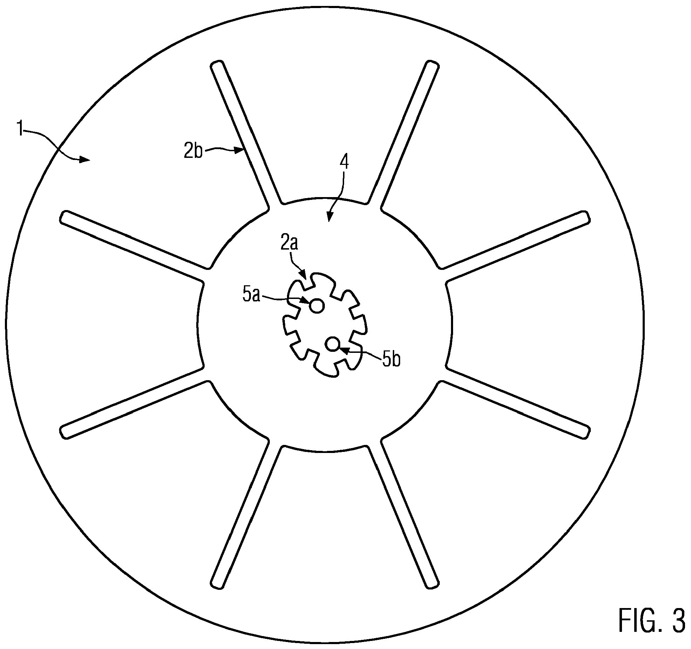

Figure 3 shows yet another alternative embodiment of the disk-shaped rotor 1. In this embodiment, the rotor has a single ring-like recess 4 that intersects all pairs of slots 2a, 2b and the rotor body is made from a transparent material.

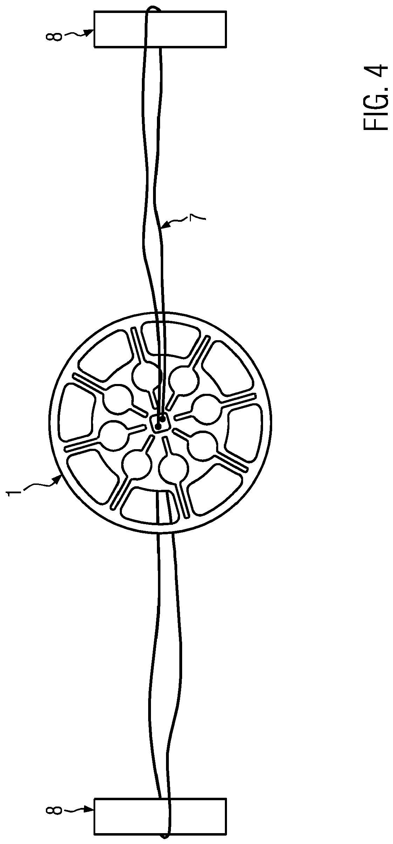

Figure 4 shows the rotor 1 of Figure 2 that is attached to a pair of strings 7 passing through the through holes 5a, 5b. The ends of the strings 7 are connected to handles 8. In Figure 3, the strings 7 are unwound.

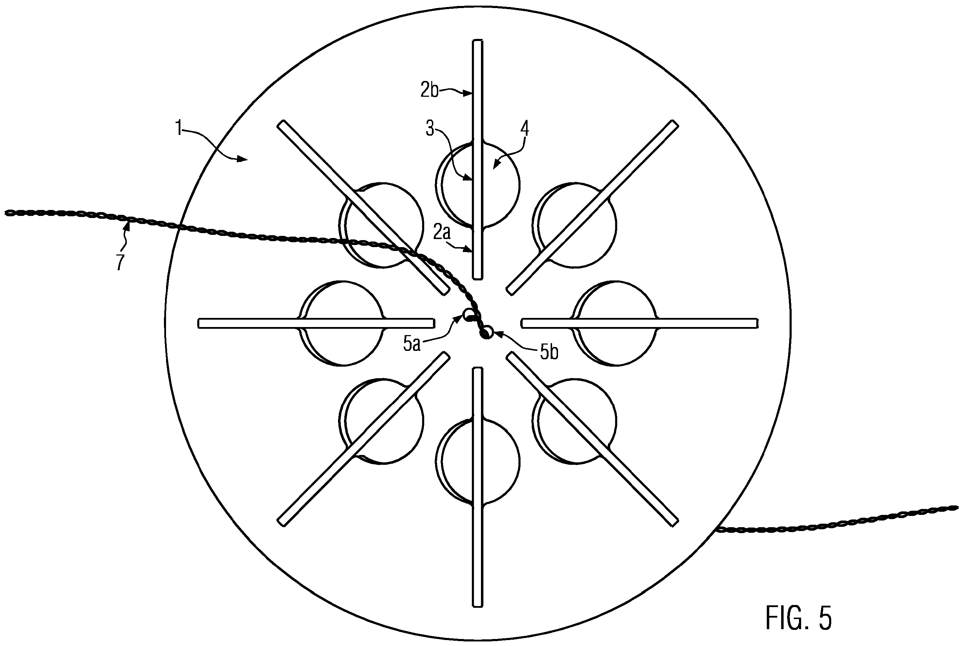

Figure 5 shows a detail view of a rotor of Figure 1 attached to a pair of strings 7 passing through the through holes 5a, 5b. In Figure 5, the strings 7 are tightly twisted and the ends of the strings are connected to handles 8 (not shown), similar to the handles shown in Figure 4. By pulling on the handles 8, the strings 7 can be unwound, thus setting the rotor 1 into a rotational motion.

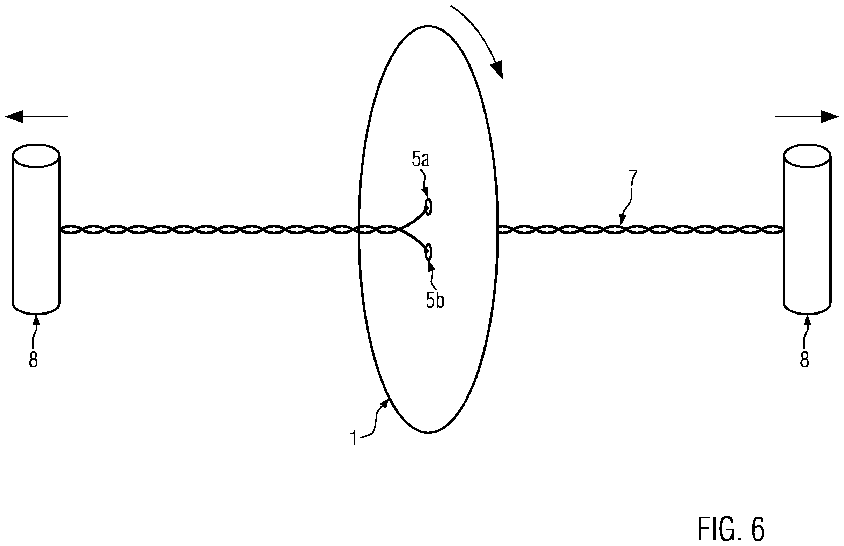

Figure 6 shows a schematic drawing illustrating the rotation mechanism of the rotor 1. By pulling the handles 8 outwards, the strings 7 are unwound and thus accelerate the rotor 1 to rotate around the axis passing through the center of the disk. The sample vessels attached to the rotor disk are omitted for clarity. The rotor itself may, for example, be formed as shown in any one of Figures 1 to 3. During rotation, the vessels are held horizontally relative to the rotational axis and point radially outwards.

## Claims

1. A system for quantifying the volume fraction of a first phase in a dispersion, comprising
a vessel for containing a sample of the dispersion,
a centrifuge for centrifuging the vessel containing the dispersion,
a computer program comprising instructions which, when the program is executed by a computer, cause the computer to carry out an image analysis algorithm on a digital photo obtained from the vessel after centrifugation.

2. The system according to claim 1, wherein the image analysis algorithm determines the size of the first phase and the size of the entire sample in the vessel and optionally calculates the volume fraction of the first phase from the ratio of the size of the first phase to the size of the entire sample in the vessel.

3. The system according to any one of the preceding claims, further comprising
a digital camera for obtaining a digital photo of the sample after centrifugation, the digital camera having an electronic interface for transferring the digital image to a computer that executes the computer program.

4. The system according to any one of the preceding claims, further comprising a sample holder for holding the vessel during centrifugation or for obtaining a digital photo of the sample after centrifugation.

5. The system according to claim 4, wherein the sample holder has at least one surface that is of a contrasting color to the color of the first phase.

6. The system according to claim 4 or 5, wherein the vessel is formed by a microfluidic channel on the sample holder.

7. The system according to any one of claims 4 to 6, wherein the sample holder is configured to hold the vessel horizontally relative to the centrifugation axis during centrifugation.

8. The system according to any one of the preceding claims, wherein the centrifuge is a hand-powered centrifuge.

9. The system according to claim 8, wherein the centrifuge is a fidget-spinner comprising two or more lobes attached to a ball bearing, the lobes being configured to rotate around the rotational axis of the ball bearing and at least one of the lobes being configured to hold the vessel during centrifugation; or wherein the centrifuge comprises a disk-shaped rotor for holding the vessel and two or more strings, the strings passing through two or more through holes disposed on the disk-shaped rotor.

10. A method for quantifying the volume fraction of a first phase in a dispersion, comprising
providing a sample of the dispersion in a vessel;
centrifuging the vessel to separate the different phases of the dispersion;
obtaining a digital photo of the sample after centrifugation;
performing a computer-implemented image analysis algorithm on the digital photo to determine the size of the first phase and the size of the entire sample in the vessel; and
calculating the volume fraction of the first phase from the ratio of the size of the first phase to the size of the entire sample in the vessel.

11. The method according to claim 10, wherein
the dispersion is an animal or human body fluid.

12. The method according to claims 10 or 11, wherein
the dispersion is animal or human blood and the first phase comprises red blood cells or the dispersion is animal or human milk and the first phase comprises cream.

13. The method according to any one of claims 10 to 12, wherein
the vessel is centrifuged using a hand-powered centrifuge.

14. The method according to any one of claims 10 to 13, wherein
during centrifugation, the vessel is placed horizontally relative to the centrifugation axis.

15. The method according to any one of claims 10 to 14, wherein
the vessel is placed on a surface that is of a contrasting color to the color of the first phase for obtaining the digital photo of the vessel.
